# FASCICULE DE BREVET EUROPEEN

(11) **EP 2 479 282 B1**
(45) Date de publication et mention de la délivrance du brevet: **28.03.2018**
(21) Numéro de dépôt: 12162356.5
(22) Date de dépôt: 29.05.2008
(51) Int. Cl.: C12Q 1/26, C07D 235/18, C07D 241/44, C07D 263/56, C07D 277/66, C12Q 1/04

(54) **Milieux comprenant des substrats enzymatiques de nitroréductase**
Medien, die enzymatische Nitroreduktasesubstrate umfassen
Media including nitroreductase enzymatic substrates

(30) Priorité: 31.05.2007 FR 0755373
(43) Date de publication de la demande: 25.07.2012
(62) Demande divisionnaire de: 08805879.7
(73) Titulaire: bioMérieux, 69280 Marcy l'Etoile (FR)
(72) Inventeur: Fabrega, Olivier, Newcastle Upon Tyne, NE3 3GJ (GB); James, Arthur, Cumbria, CA13 9UX (GB); Orenga, Sylvain, 01160 Neuville Sur Ain (FR); Salwatura, Vindhya Lakshika, Newcastle Upon Tyne, NE4 9JB (GB); Stanforth, Stephen, NE43 7 EH Northumberland (GB)
(74) Mandataire: Bitaud, Valérie Marie-Odile

(56) Documents cités:
- WO-A-00/28073
- WO-A1-93/04077
- WO-A2-2004/058787
- TEMIZ O ET AL: "Synthesis and microbiological activity of some novel 5- or 6-methyl-2-(2,4-disubstituted phenyl) benzoxazole derivatives", FARMACO, vol. 53, no. 5, 30 mai 1998 (1998-05-30), pages 337-341, XP009094797, LAUSANNE ISSN: 0014-827X
- BOUGRIN K. ET AL.: "Trois Nouvelles Voies de Synthèse des Dérivés 1,3-Azoliques Sous Micro-ondes", TETRAHEDRON, vol. 54, no. 28, 9 juillet 1998 (1998-07-09), pages 8055-8064, XP004123999, UNITED KINGDOM ISSN: 0040-4020
- CHAIGNON P ET AL: "Purification and identification of a Bacillus nitroreductase: Potential use in 3,5-DNBTF biosensoring system", ENZYME AND MICROBIAL TECHNOLOGY, vol. 39, no. 7, 3 novembre 2006 (2006-11-03), pages 1499-1506, XP005661982,

## Description

La présente invention concerne la détection d'activité nitroréductase, au moyen de substrats enzymatiques particuliers. Ces substrats enzymatiques sont utilisables dans les applications comportant une étape de réduction enzymatique produisant un signal physico-chimique notamment en microbiologie, biochimie, immunologie, biologie moléculaire, histologie, etc.

Il existe actuellement de très nombreux milieux permettant la détection de microorganismes. Cette détection peut être basée notamment sur l'utilisation de substrats particuliers, spécifiques d'une enzyme du microorganisme que l'on souhaite détecter. D'une manière générale, les substrats synthétiques d'enzymes sont constitués d'une première partie spécifique de l'activité enzymatique à révéler, et d'une seconde partie faisant office de marqueur, généralement chromogène ou fluorescent. Ainsi dans le cas des bactéries, par le choix des substrats, selon qu'il y a réaction ou non, il est possible de caractériser la nature d'un microorganisme. Une activité nitroréductase peut notamment être utilisée pour révéler un groupe, un genre ou une espèce de bactéries. Il peut également être utilisé pour suivre le métabolisme réducteur de microorganismes, par exemple lié à leur croissance ou à l'inhibition de cette croissance.
La capacité de certaines bactéries de réduire les composés nitro-aromatiques est connue depuis de nombreuses années. Asnis (1957) a rapporté l'isolation d'une flavoproteine à partir d'extraits d'*E. coli* qui était capable de réduire l'acide p-nitrobenzoique. Depuis ce rapport, l'activité nitroaryl-réductase a été identifiée dans diverses variétés d'organismes. Ceci inclut des aérobies strictes tels que *Pseudomonas spp.* (Won et al. 1974) et *Nocardia spp.* (Villanueva 1964), des anaérobies strictes tels que *Clostridium spp.* (Ancermaier & Simon 1983) et *Veillonella spp.* (McCormick et al. 1976), ou encore des champignons (Masuda & Ozaki 1993) et des parasites eucaryotes (Douch 1975). Il existe une gamme de substrats qui ont été désignées comme étant susceptibles d'être réduits par des nitroaryl-réductases bactériennes. Il s'agit surtout de composés nitro-aromatiques tels que l'acide p-nitrobenzoique, le p-nitrophénol, la p-nitroaniline et le 2,4, 6-trinitrotoluène (McCormick et al. 1976).

D'une manière générale, la détection de l'activité enzymatique nitroréductase est réalisée par des méthodes indirectes telles que le suivi de la disparition du substrat ou d'un cofacteur. Par exemple, Kitamura et al. (1983) ont étudié la réduction du méthyl p-nitrobenzoate et d'une gamme d'autres composés aromatiques nitrés avec des extraits d'*E*. *coli.* Toutefois, cette méthode est peu sensible et n'est pas adaptée à une détection en milieu hétérogène. On peut citer également la demande WO 00/28073 qui décrit un substrat fluorogène à base de nitrocoumarine pour la détection directe des activités nitroaryl-réductases. Ce type de composé nitro-aromatique est capable de produire, après réduction, un composé très fluorescent qui est donc facilement détectable. Toutefois ce substrat n'est pas bien adapté à une détection en milieu hétérogène.
La présente invention se propose donc d'améliorer les substrats de nitroréductase permettant la détection de microorganismes. Comparativement aux substrats existants, ces nouveaux substrats sont de synthèse aisée, et peuvent être utilisés notamment en milieux gélifiés pour la détection de micro-organismes car ils produisent une coloration ne diffusant pas dans le milieu réactionnel.

Avant d'aller plus avant dans la description de l'invention, les définitions ci dessous sont données afin de faciliter l'exposé de l'invention.
Par substrat enzymatique, on entend un substrat pouvant être modifié par une enzyme en un produit permettant la détection, directe ou indirecte d'un microorganisme, d'une cellule ou d'une organelle. Dans le cas des substrats de nitroréductase, ce substrat comprend notamment une fonction nitrate qui est partiellement ou totalement réduite par l'activité enzymatique à révéler, la réduction de cette fonction nitrate modifiant certaines propriétés physicochimiques de la molécule, permettant de suivre cette réduction.
Les substrats mis en oeuvre selon l'invention sont particulièrement bien adaptés à une utilisation en cytométrie en flux, car le produit de la réduction restant principalement localisé dans la cellule exprimant l'activité enzymatique, il est possible de compter spécifiquement les cellules exprimant cette activité, voire de les séparer du reste de l'échantillon.

Les substrats mis en oeuvre selon l'invention sont également bien adaptés à une utilisation en histoenzymologie, car le produit de la réduction restant principalement localisé sur le lieu de la réduction, il est possible d'identifier spécifiquement les cellules ou organelles exprimant cette activité au sein d'un tissu.
Du fait de leur faible toxicité, les substrats mis en oeuvre selon l'invention sont bien adaptés au suivi d'activité nitroréductase de culture cellulaire.
Les substrats mis en oeuvre selon l'invention sont également très bien adaptés à une utilisation en milieu de détection et/ou d'identification car ils produisent une coloration ou une fluorescence ne diffusant pas dans le milieu réactionnel. Dans la présente demande, le terme coloration est employé pour couvrir une coloration dans le spectre visible, absorption de lumière, ou une fluorescence, absorption à une longueur d'onde (λₑₓ) et émission à une longueur d'onde supérieure (λₑₘ).
Les substrats mis en oeuvre selon l'invention peuvent être salifiés, c'est à dire sous forme de sel tel que chlorure, bromure, potassium ou trifluoroacétate.
Par nitroréductase, on entend une enzyme pouvant réduire totalement ou partiellement un groupement NO₂.
Par groupement alkyle, on entend une chaîne de groupements hydrocarbonés saturés, tel que notamment un alkyle en C₁-C₆, c'est à dire un alkyle droit ou ramifié ayant de 1 à 6 atomes de carbone. A titre d'exemple, on peut citer le méthyle, l'éthyle, le propyle, l'iso-propyle, le butyle, le t-butyle, le pentyle, l'iso-pentyle et l'hexyle.
Par groupement aryle, on entend un groupement fonctionnel (ou substituant) qui dérive d'un noyau aromatique tel que notamment un noyau aromatique en C6-C10, notamment phényle, benzyle, 1-naphtyle ou 2-naphtyle.
Par groupement carboxyle, on entend notamment un groupe fonctionnel composé d'un atome de carbone, lié par une double liaison à un premier atome d'oxygène, et par une liaison simple à un second atome d'oxygène, lui-même chargé négativement ou relié à un atome d'hydrogène. En fonction du pKₐ de la molécule et du pH du milieu, le groupement carboxyle peut être sous forme ionisée, c'est à dire sans H lié au second atome d'oxygène, qui est alors chargé négativement.
Par milieu réactionnel, on entend un milieu comprenant tous les éléments nécessaires à l'expression d'un métabolisme et/ou à la croissance de microorganismes, d'une cellule ou d'une organelle. Ce milieu réactionnel peut être utilisé en cytométrie en flux, histoenzymologie, culture cellulaire ... ou en tant que milieu de détection et/ou d'identification de microorganismes.
Le milieu réactionnel peut être solide, semi-solide ou liquide. Par milieu solide, on entend par exemple un milieu gélifié. L'agar est l'agent gélifiant traditionnel en microbiologie pour la culture des microorganismes, mais il est possible d'utiliser de la gélatine ou de l'agarose. Un certain nombre de préparation sont disponibles dans le commerce, comme par exemple l'agar Columbia, la gélose Trypcase-soja, la gélose Mac Conkey, la gélose Sabouraud ou plus généralement celles décrites dans le Handbook of Microbiological Media (CRC Press).
Le milieu réactionnel peut comprendre un ou plusieurs éléments en combinaison, tels que des acides aminés, des peptones, des hydrates de carbone, des nucléotides, des minéraux, des vitamines, des antibiotiques, des tensioactifs, des tampons, des sels de phosphate, d'ammonium, de sodium, de métaux.
Le milieu peut comprendre également un colorant. A titre indicatif, on peut citer comme colorant le bleu d'Evans, du rouge neutre, du sang de mouton, du sang de cheval, un opacifiant tel que l'oxyde de Titane, de la nitroaniline, du vert malachite, du vert brillant...
Le milieu réactionnel peut être un milieu de détection et/ou d'identification, c'est à dire un milieu de révélation, ou un milieu de culture et de révélation. Dans le premier cas, la culture des microorganismes est effectuée avant ensemencement et, dans le deuxième cas, le milieu de détection et/ou d'identification constitue également le milieu de culture. Par échantillon biologique, on entend un échantillon clinique, issu d'un prélèvement de liquide biologique ou un échantillon alimentaire, issu de tout type d'aliment. Cet échantillon peut être ainsi liquide ou solide et on peut citer d'une manière non limitative, un échantillon clinique de sang, de plasma, d'urine, de fécès, de prélèvement de nez, de gorge, de peau, de plaie, de liquide céphalo-rachidien, un échantillon alimentaire d'eau, de boisson tels que le lait, un jus de fruits; de yaourt, de viande, d'oeuf, de légume, de mayonnaise, de fromage ; de poisson..., un échantillon alimentaire issu d'une alimentation destinée aux animaux, tel que notamment un échantillon issu de farine animale. L'échantillon peut également être un prélèvement de l'environnement clinique, échantillon issu de farine animale. L'échantillon peut également être un prélèvement de l'environnement clinique, d'élevage ou de production alimentaire, cosmétique ou pharmaceutique. Par prélèvement d'environnement, on entend notamment un prélèvement de surface, de liquide, de matière première ou de produit.
Au sens de la présente invention, le terme microorganisme recouvre les bactéries, notamment à gram négatif et à gram positif, les levures, les parasites, les champignons, et plus généralement, les organismes généralement unicellulaire, invisibles à l'oeil nu, qui peuvent être multipliés et manipulés en laboratoire.
A titre de bactéries à Gram négatif, on peut citer les bactéries des genres suivants : *Pseudomonas*, *Escherichia*, *Salmonella, Shigella, Enterobacter*, *Klebsiella*, *Serratia*, *Proteus*, *Campylobacter, Haemophilus*, *Morganella*, *Vibrio, Yersinia*, *Acinetobacter*, *Branhamella, Neisseria*, *Burkholderia, Citrobacter, Hafnia*, *Edwardsiella, Aeromonas, Moraxella*, *Pasteurella*, *Providencia*, *Actinobacillus*, *Alcaligenes*, *Bordetella*, *Cedecea*, *Erwinia*, *Pantoea*, *Ralstonia*, *Stenotrophomonas*, *Xanthomonas et Legionella.*
A titre de bactéries à Gram positif, on peut citer les bactéries des genres suivants : *Aerococcus*, *Enterococcus*, *Streptococcus*, *Staphylococcus*, *Bacillus*, *Lactobacillus*, *Listeria*, *Clostridium*, *Gardnerella, Kocuria*, *Lactococcus*, *Leuconostoc*, *Micrococcus*, *Falkamia, Gemella, Pediococcus, Mycobacterium* et *Corynebacterium.*
A titre de levures, on peut citer les levures des genres suivants: *Candida*, *Cryptococcus*, *Saccharomyces* et *Trichosporon*.

A ce titre, l'invention concerne une utilisation d'un substrat enzymatique de formule (I) suivante : 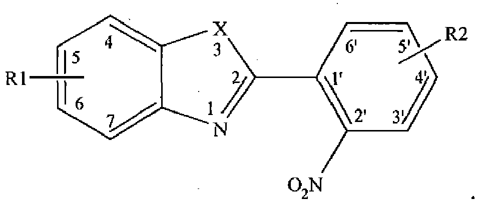 selon laquelle
- X représente NX1; O, NX1-CO,
- R1 représente rien, ou un substituant choisi parmi Cl, CH₃, Br, F, I, alkyle, aryle, carboxyle
- R2 représente rien ou un substituant choisi parmi Cl ; O-CH₂-O ; O-CH₃ ; F, diéthylènediamine-CH₃, NR3R4, Br, I, alkyle, aryle, carboxyle, NO₂ 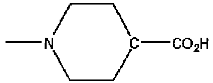
- R3 et R4 représentent indépendamment H ou un groupement alkyle ayant de 1 à 4 atomes de carbone
- X1 est choisi parmi H, CH₃, C₂H₄Ph, OH, alkyle, aryle
pour la détection chez des microorganismes, d'une activité nitroréductase.
La liaison entre l'Azote en 1 et le Carbone en 2 de l'hétérocycle peut être simple (1,2-Dihydro) ou double, préférentiellement double.
Bien entendu, le substrat utilisé selon l'invention peut comprendre plusieurs substituants, c'est-à-dire plusieurs groupements R1, R2 en différente position du cycle. Lorsque R2 représente O-CH₂-O, R2 représente un cycle accolé au nitrophénol, deux de leurs sommets étant partagés. Lorsque R1 et/ou R2 est un groupement alkyle ou aryle, il peut également être substitué par un ou plusieurs substituants tel que OH, carboxyle, Br, Cl, F, I.
Lorsque X représente NX1-CO, le cycle comprenant X est préférentiellement un cycle à 6 sommets, comprenant NX1 en position 3 et un C relié par une double liaison à un O en position 4 dudit cycle à 6 sommets.
Selon un mode préféré de réalisation de l'invention, X représente N-X1.
Selon un autre mode préféré de réalisation de l'invention, X représente O.
Selon un autre mode préféré de réalisation de l'invention, X représente NX1-CO. Selon un mode préféré de réalisation de l'invention, R1 est en position 5.
Selon un mode préféré de réalisation de l'invention, R2 est en position 4'. Selon un autre mode préféré de réalisation de l'invention, R2 est en position 5'. Selon un autre mode préféré de réalisation de l'invention, R2 est en position 4' et en position 5'.
Selon un mode particulier de réalisation de l'invention, ledit milieu réactionnel comprend au moins un substrat enzymatique qui répond à la formule (I) ci dessus, selon laquelle :
- R1 et R2 ne représentent rien

Selon un mode particulier de réalisation de l'invention, ledit substrat enzymatique répond à la formule (I) ci dessus, selon laquelle :
- X représente O
- R1 représente Cl, préférentiellement en position 5
- R2 ne représente rien

Selon un mode particulier de réalisation de l'invention, ledit substrat enzymatique répond à la formule (I) ci dessus, selon laquelle :
- X représente O
- R1 représente CH₃, préférentiellement en position 5
- R2 ne représente rien

Selon un mode particulier de réalisation de l'invention, ledit substrat enzymatique répond à la formule (I) ci dessus, selon laquelle :
- X représente O
- R1 ne représente rien
- R2 représente Cl, préférentiellement en position 5'

Selon un mode particulier de réalisation de l'invention, ledit substrat enzymatique répond à la formule (I) ci dessus, selon laquelle :
- X représente O
- R1 ne représente rien
- R2 représente O-CH₂-O, préférentiellement accolé au nitrophénol en position 4' et 5'

Selon un mode particulier de réalisation de l'invention, ledit substrat enzymatique répond à la formule (I) ci dessus, selon laquelle :
- X représente O
- R1 ne représente rien
- R2 représente O-CH₃, préférentiellement en position 4' et 5'

Selon un mode particulier de réalisation de l'invention, ledit substrat enzymatique répond à la formule (I) ci dessus, selon laquelle :
- X représente O
- R1 ne représente rien
- R2 représente F, préférentiellement en position 5'

Selon un mode particulier de réalisation de l'invention, ledit substrat enzymatique répond à la formule (I) ci dessus, selon laquelle :
- X représente O
- R1 ne représente rien
- R2 représente diéthylènediamine-CH₃, préférentiellement en position 5'

Selon un mode particulier de réalisation de l'invention, ledit substrat enzymatique répond à la formule (I) ci dessus, selon laquelle :
- X représente NX1
- X1 représente CH₃, préférentiellement en position 3
- R1 ne représente rien
- R2 ne représente rien

Selon un mode particulier de réalisation de l'invention, ledit substrat enzymatique répond à la formule (I) ci dessus, selon laquelle :
- X représente NX1
- X1 représente CH₃
- R1 ne représente rien
- R2 représente Cl, préférentiellement en position 5'

Selon un mode particulier de réalisation de l'invention, ledit substrat enzymatique répond à la formule (I) ci dessus, selon laquelle :
- X représente NX1
- X1 représente CH₃
- R1 ne représente rien
- R2 représente O-CH₂-O, préférentiellement accolé au nitrophénol en position 4' et 5'

Selon un mode particulier de réalisation de l'invention, ledit substrat enzymatique répond à la formule (I) ci dessus, selon laquelle :
- X représente NX1
- X1 représente C₂H₄Ph
- R1 ne représente rien
- R2 ne représente rien

Selon un mode particulier de réalisation de l'invention, ledit substrat enzymatique répond à la formule (I) ci dessus, selon laquelle :
- X représente NX1
- X1 représente C₂H₄Ph
- R1 ne représente rien
- R2 représente Cl, préférentiellement en position 5'

Selon un mode particulier de réalisation de l'invention, ledit substrat enzymatique répond à la formule (I) ci dessus, selon laquelle :
- X représente NX1
- X1 représente C₂H₄Ph
- R1 ne représente rien
- R2 représente O-CH₂-O, préférentiellement accolé au nitrophénol en position 4' et 5'

Selon un mode particulier de réalisation de l'invention, ledit substrat enzymatique répond à la formule (I) ci dessus, selon laquelle :
- X représente NX1-CO
- X1 représente H
- R1 et R2 ne représentent rien

Selon un mode particulier de réalisation de l'invention, ledit substrat enzymatique répond à la formule (I) ci dessus, selon laquelle :
- X représente NX1-CO
- X1 représente CH₃
- R1 et R2 ne représentent rien

Selon un mode particulier de réalisation de l'invention, ledit substrat enzymatique répond à la formule (I) ci dessus, selon laquelle :
- X représente NX1-CO
- X1 représente CH₃
- R1 ne représente rien
- R2 représente Cl, préférentiellement en position 5'

Selon un mode particulier de réalisation de l'invention, ledit substrat enzymatique répond à la formule (I) ci dessus, selon laquelle :
- X représente NX1
- X1 représente H
- R1 et R2 ne représentent rien

Selon un mode particulier de réalisation de l'invention, ledit substrat enzymatique répond à la formule (I) ci dessus, selon laquelle :
- X représente NX1-CO
- X1 représente H
- R1 représente ne représente rien
- R2 représente Cl en position 5'

Selon un mode particulier de réalisation de l'invention, ledit substrat enzymatique répond à la formule (I) ci dessus, selon laquelle :
- X représente O
- R1 ne représente rien
- R2 représente NO₂, préférentiellement en position 4'

Préférentiellement, le substrat mis en oeuvre dans un milieu selon l'invention est choisi parmi les substrats décrit dans le tableau 1.

**Tableau 1 - Exemples de substrats mis en oeuvre dans un milieu selon l'invention**

| **Substrats** | **Référence** | **X** | **X1** | **R1** | **R2** |
|---|---|---|---|---|---|
| 2-(2'-Nitrophényl)-benzoxazole | Substrat 1 | O | - | - | - |
| | 64 | | | | |
| 2-(2'-Nitrophényl)-5-chloro-benzoxazole | Substrat 2 | O | - | Cl | - |
| | VLS259, 72 | | | | |
| 2-(2'-Nitrophényl)-5-méthyl-benzoxazole | Substrat 3 | O | - | CH₃ | - |
| | VLS271, 75 | | | | |
| 2-(2'-Nitro-5'-chloro-phényl)-benzoxazole | Substrat 4 | O | - | - | Cl |
| | VLS269, 71 | | | | |
| 2-(2'-Nitro-4',5'-méthylènedioay-phényl)- | Substrat 5 | O | - | - | O-CH₂-O |
| 2-(2'-Nitro-4',5'-méthylènedioxy-phényl)-benzoxazole | Substrat 5 | O | - | - | O-CH₂-O |
| | 70 | | | | |
| 2-(2'-Nitro-4',5'-diméthoxy-phényl)-benzoxazole | Substrat 6 | O | - | - | O-CH₃, O-CH₃ |
| | VLS261, 73 | | | | |
| 2-(2'-Nitro-5'-fluoro-phényi)-benzoxazole | Substrat 7 | O | - | - | F |
| | OF20B | | | | |
| 2-(2'-Nitro-5'-(N-méthylpipérazinyl)-phényl)-benzoxazole | Substrat 8 | O | - | - | Diéthylenediamine-CH₃ |
| | OF29A | | | | |
| 2-(2'-Nitrophényl)-3-méthyl-benzimidazole | Substrat 9 | NX1 | CH₃ | - | - |
| | 40 | | | | |
| 2-(2'-Nitro-5'-chloro-phényl)-3-méthy-benzimidazole | Substrat 10 | NX1 | CH₃ | - | Cl |
| | 44 | | | | |
| 2-(2'-Nitro-4',5'-méthylènedioxy-phényl)-3-méthyl-benzimidazole | Substrat 11 | NX1 | CH₃ | - | O-CH₂-O |
| | 45 | | | | |
| 2-(2'-Nitrophényl)-3-phénéthyl-benzimidazole | Substrat 12 | NX1 | C₂H₄Ph | - | - |
| | 54 | | | | |
| 2-(2'-Nitro-5'-chloro-phényl)-3-phénéthyl-benzimidazole | Substrat 13 | NX1 | C₂H₄Ph | - | Cl |
| | 61 | | | | |
| 2-(2'-Nitro-4',5'-méthylènedioxy-phényl)-3-phénéthyl-benzimidazole | Substrat 14 | NX1 | C₂H₄Ph | - | O-CH₂-O |
| | 62 | | | | |
| 2-(2'-Nitro-5'-fluoro-phényl)-benzothiazole | Substrat 15 | S | - | - | F |
| | OF26A | | | | |
| 2-(2'-Nitrophényl)-quinoxazol-4-one | Substrat 16 | NX1-CO | H | - | - |
| 2-(2'-Nitrophényl)-3-méthyl-quinoxazol-4-one | Substrat 17 | NX1-CO | CH₃ | - | - |
| 2-(2'-Nitro-5'-chloro-phényl)-3-méthyl-quinoxazol-4-one | Substrat 18 | NX1-CO | CH₃ | - | Cl |
| 2-(2'-Nitrophényl)-benzimidazole | Substrat 19 | NX1 | H | - | - |
| 2-(2'-Nitro-5'-chloro-phényl)-quinoxazol-4-one | Substrat 20 | NX1-CO | H | - | Cl |
| 2-(2'-Nitro-5'-chloro-phényl)-benzothiazole | Substrat 21 | S | - | - | Cl |
| | CVX-16 | | | | |
| 2-(2'-Nitrophényl)-benzothiazole | Substrat 22 | S | - | - | - |
| 2-(2'-Nitro-5'-(N-méthylpipérazinyl)-phényl)-benzothiazole | Substrat 23 | S | - | - | Diéthylènediamine-CH₃ |
| 2-(2'-Nitro-5'-N-(carboxylpypéridinyl)-phényl)-benzothiazole | Substrat 24 | S | - | - | 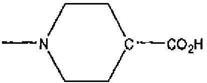 |
| 2-(2',4'-Dinitrophényl)benzoxazole | Substrat 25 | O | | | NO₂ |
| | OF91C | | | | |
| 2-(2'-Nitrophényl)-1,2,-Dihvdrobenzothiazole | Substrat 26 | S | - | - | - |
| 2-(2'-Nitro-5'-fluoro-phényl)-1,2,-Dihydrobenzothiazole | Substrat 27 | S | - | - | F |

L'invention concerne également une utilisation d'un substrat de formule (I) tel que défini ci-dessus, dans un milieu réactionnel, qui est un milieu de détection et/ou d'identification de microorganismes. Préférentiellement, ledit substrat dans ledit substrat dans ledit milieu est à une concentration comprise entre 1 et 1000 mg/l, préférentiellement entre 10 et 500 mg/l.
Selon un mode particulier de réalisation de l'invention, ledit milieu comprend en outre au moins un autre substrat enzymatique, spécifique d'une activité enzymatique différente de celle détectée par le substrat présent dans le milieu selon l'invention.
L'hydrolyse enzymatique du ou des autres substrats génère un signal détectable, différent du signal détecté par le substrat de l'invention, comme par exemple des produits colorés ou fluorescents différents, pour permettre la mise en évidence comme la détection et/ou l'identification et/ou la quantification d'un ou plusieurs microorganismes. A titre d'autre substrat spécifique, on peut utiliser tout autre substrat classiquement utilisé dans la détection des microorganismes. La concentration de l'autre substrat enzymatique spécifique est généralement comprise entre 0,01 et 1 g/l. L'homme du métier pourra déterminer facilement une telle concentration en fonction du substrat utilisé. A titre indicatif, il est possible de combiner les substrats selon l'invention avec des substrats enzymatiques de peptidase, d'osidase, d'estérase ou de réductase. En particulier, il est possible d'associer un substrat selon l'invention avec un substrat d'osidase, tel que le 5-Bromo-4-chloro-3-indolyl-β-glucoside, ou l'Alizarine-β-galactoside ou avec un substrat d'estérase, tel que le 5-Bromo-6-chloro-3-indoxyl-octanoate ou le 5-Bromo-3-indoxyl-phosphate.
Selon un mode particulier de réalisation de l'invention, ledit milieu comprend en outre au moins un autre substrat enzymatique spécifique de l'activité enzymatique détectée par le substrat présent dans le milieu selon l'invention.
Par le choix particulier de substrats, il est alors possible d'identifier des groupes de microorganismes exprimant une même activité enzymatique. La concentration de l'autre substrat enzymatique spécifique est généralement comprise entre 0,01 et 1 g/l. L'homme du métier pourra déterminer facilement une telle concentration en fonction du substrat utilisé. En particulier, il est possible d'associer un substrat selon l'invention à un substrat fluorogène à base de nitrocoumarine tel que décrit dans la demande WO 00/28073.

L'invention concerne également l'utilisation des substrats enzymatiques de formule (I), tels que définis ci dessus ou d'un milieu de détection et/ou d'identification tel que défini

L'invention concerne également l'utilisation des substrats enzymatiques de formule (I), tels que définis ci-dessus ou l'utilisation d'un substrats enzymatiques de formule (I) dans un milieu de détection et/ou d'identification telle que définie ci-dessus pour la détection chez des micro-organismes d'au moins une activité nitroréductase.
L'invention concerne également un procédé pour la détection chez des micro-organismes d'au moins une activité nitroréductase, caractérisé en ce qu'il comprend ou est constitué par les étapes suivantes :
a) disposer d'un milieu de détection et/ou d'identification tel que défini ci dessus,
b) ensemencer le milieu avec un échantillon biologique à tester,
c) laisser incuber, et
d) révéler la présence d'au moins une activité nitroréductase
L'ensemencement des microorganismes peut être réalisé par toutes les techniques d'ensemencement connues de l'homme du métier. Une étape d'incubation peut être réalisée à une température pour laquelle l'expression de l'activité enzymatique que l'on souhaite détecter est optimale, que l'homme du métier peut choisir aisément selon l'activité enzymatique et les microorganismes à détecter. L'étape d) peut s'effectuer par un examen visuel, par colorimétrie ou fluorimétrie. Lors de l'étape d), on peut révéler la présence de l'activité nitroréductase, seule ou en combinaison avec d'autres activités enzymatiques.

Les exemples ci-dessous sont donnés à titre explicatif et n'ont aucun caractère limitatif. Ils permettront de mieux comprendre l'invention.

### EXEMPLES

### 1 - Synthèse de substrats

### • X est S

### Substrat 22 : 2-(2'-Nitrophényl)-benzothiazole

Un mélange de 1éq de 2-Aminophénol, d'éthanol et de 1éq de 2-Nitrobenzaldéhyde a été chauffé par un bain d'huile à reflux à la température de 110°C durant une heure.

Le mélange a été refroidi à température ambiante et le précipité a été récupéré par filtration. Afin de récupérer un maximum de produit, le ballon a été rincé avec le filtrat pour évaporé une partie significative.

Le solide a été séché dans un dessiccateur sous pression réduite pour obtenir le produit intermédiaire.

Le solide précédemment obtenu a été dissout dans 100 ml de Dichlorométhane, puis a été ajouté petit à petit 1éq de 2,3-Dicyano-5,6-dichloro-parabenzoquinone à température ambiante. La réaction s'est déroulée durant la nuit, la verrerie a été filtrée et rincée avec du D.C.M. afin de récupérer le maximum de produit puisque il est soluble dans le D.C.M.

Le solvant a été évaporé sous pression réduite afin d'obtenir le produit.

### Substrat 15 : 2-(2'-Nitro-5'-fluoro-phényl)-benzothiazole

Un mélange de 3,00g (0,024mol) de 2-Aminothiophénol et de 4,05g (0,024mol) de 5-Nitro-3-fluoro benzaldéhyde dans l'éthanol (150ml) a été chauffé à reflux durant 2 heures. Apres avoir été refroidit à température ambiante, la solution a été concentrée sous pression réduite. Le solide restant a été dilué dans un mélange d'eau et d'acétate d'éthyle (500ml), et la phase aqueuse a été extraite encore deux fois en utilisant de l'acétate d'éthyle (2x50ml). Les phases organiques réunies on été séchées (MgSO₄), filtrées et concentrées afin d'obtenir le produit brut, qui a été recristallisé à partir d'éthanol. Une masse de 1,28g de fins cristaux rouges d'OF54A2 a été obtenue avec un rendement de 47% et un point de fusion mesuré de 156-158°C.

Les données qui ont été obtenues en RMN sont indiquées ci dessous :
¹H NMR (270 MHz, CDCl₃): δ = 6.87 (1H, t, *J* = 4 Hz, Ar-*H*), 6.89 (1H, s, Ar-*H*), 7.05 (1H, t, *J* = 4 Hz, Ar-*H*), 7.11 (1H, d, *J* = 3 Hz, Ar-*H*), 7.13 (1H, d, *J* = 3 Hz, Ar-*H*), 7.69 (1H, dd, *J* = 9 Hz, Ar-*H*), 8.17 (1H, dd, *J* = 9 Hz, Ar-*H*).

### Substrat 23 : 2-(2'-Nitro-5'-(N-méthylpipérazinyl)-phényl)-benzothiazole_(OF80A)

0,31g (0,0030mol) d'N-Méthylpipérazine a été ajouté au mélange de 0,41g (0,015mol) de 2-(2'-Nitro-5'-fluorophényl)-benzothiazole et de 0,32g (0,0023mol) de K₂CO₃ dans 5 ml de Diméthysulfoxide. La réaction a été laissée sous agitation magnétique et chauffée à 90°C, en vérifiant son avancement par CCM.

Après achèvement de la réaction (au moins 8 heures) le mélange réactionnel a été dilué dans de l'acétate d'éthyle (77mL), la phase organique ainsi obtenue a été soigneusement lavée avec de 2 fois 65ml d'eau, puis avec 65ml de saumure et séchée à l'aide de sulfate de sodium afin d'obtenir 0,36g d'un solide jaune orangé très électrostatique, OF80A avec un rendement de 68%. On peut effectuer une purification par chromatographie sur colonne si nécessaire, cela n'a pas été le cas pour nous.
¹H NMR (270 MHz, CDCl₃): δ = 2.35 (3H, s, C*H₃*), 2.45 (2H, t, *J* = 7 Hz, C*H₂*-CH₂), 3.49 (2H, t, *J* = 7 Hz, C*H₂*-CH₂), 6.85 (1H, d, *J* = 7 Hz, Ar-*H*), 6.90 (1H, t, *J* = 7 Hz, Ar-*H*), 7.41 (1H, d, *J* = 7 Hz, Ar-*H*), 7.43 (1H, d, *J* = 7 Hz, Ar-*H*), 7.85 (1H, d, *J* = 7 Hz, Ar-*H*), 8.05 (1H, t, *J* = 7 Hz, Ar-H).
LC-MS-DI m/z Found: 355.57 (C₁₈H₁₈N₄O₂S) (M+H): requires M. 354.43.

### Substrat 21 : 2-(2'-Nitro-5'-chloro-phényl)-benzothiazole

Le substrat No 21 a été synthétisé de façon analogue à la synthèse du 2-(2'-Nitro-5'-fluoro-phényl)-benzothiazole (substrat No 15).

### • X est O

### Substrat 1 - 2-(2'-Nitrophényl)-benzoxazole

Un mélange de 8,73g (0,0800 mol) de 2-Aminophénol, de 20 ml d'éthanol et de 12,09g (0,0800 mol) de 2-Nitrobenzaldéhyde a été chauffé par un bain d'huile à reflux à la température de 110°C durant une heure.

Le mélange a été refroidi à température ambiante et le précipité a été récupéré par filtration. Afin de récupérer un maximum de produit, le ballon a été rincé avec le filtrat pour évaporer une partie significative.

Le solide a été séché dans un dessiccateur sous pression réduite pour obtenir 13,25g de 2-(2'-Nitro-benzylidineamino)-phénol avec un rendement de 69%.

Le solide précédemment obtenu a été dissous dans 100ml de dichlorométhane, puis a été ajouté petit à petit 12,43g (0,0547 mol) de 2,3-Dicyano-5,6-dichloro-parabenzoquinone à température ambiante. La réaction s'est déroulée pendant une nuit. La verrerie a été filtré, puis rincée avec du D.C.M. afin de récupérer le maximum de produit puisque il est soluble dans le D.C.M.

Le solvant a été évaporé sous pression réduite afin d'obtenir 8,39g de 2-(2-Nitrophényl)-benzoxazole, un solide marron-beige avec un rendement de 64%. Les données qui ont été obtenues en RMN sont indiquées ci dessous :
¹H NMR (270 MHz, CDCl₃): δ = 7.40 (1H, t, *J* = 5 Hz, Ar-*H*), 7.42 (1H, t, *J* = 5 Hz, Ar-*H*), 7.50 (1H, d, *J* = 7 Hz, Ar-*H*), 7.72 (1H, t, *J* = 5 Hz, Ar-*H*), 7.73 (1H, t, *J* = 5 Hz, Ar-*H*), 7.80 (1H, d, *J* = 7 Hz, Ar-H), 7.89 (1H, d, *J* = 7 Hz, Ar-*H*), 8.15 (1H, d, *J* = 7 Hz, Ar-*H*).

### Substrat 2 - 2-(2'-Nitrophényl)-5-chloro-benzoxazole - VLS259

Le substrat No 2 a été synthétisé de façon analogue à la synthèse du 2-(2'-Nitrophényl)-benzoxazole (substrat No 1).

### Substrat 6 - 2-(2'-Nitro-4',5'-dimethoxy-phényl)-benzoxazole - VLS261

### 1. Synthèse du 2-((3'-Chloro-6'-nitro-benzylidene)-amino)-phénol (VLS 263)

Un mélange de 2,18g (0,02 mol) de 2-Hydroxyaniline et 3,73g (0,02 mol) de 2-Nitro-5-chlorobenzaldéhyde dans l'éthanol a été chauffé à reflux durant 3-4 heures. Le précipité cristallisé résultant a été collecté et séché dans un dessicateur à pression réduite. Une masse de 1,98g de fin cristaux oranges a été obtenue avec un rendement de 80% et un point de fusion mesuré de 144-146°C.

Les données qui ont été obtenues en RMN sont indiquées ci dessous :
¹H-NMR:(CDCl₃) δ 6.95 (1H, t, J=8 Hz, Ph-*H*), 7.05 (1H, dd, J=8 Hz, J=1.2 Hz, Ph-*H*), 7.10 (1H, s (broad), O*H*), 7.28 (1H, t, J=8 Hz, Ph-*H*), 7.33 (1H, dd, J=8 Hz, J= 1.4 Hz, Ph-*H*), 7.60 (1H, dd, J=8.66 Hz, J=2.2 Hz, 4'*H*), 8.05 (1H, d, J=8.66 Hz, 5'*H*), 8.23 (1H, d, J=2.4 Hz, 2'*H*), 9.27 (1H, s, =C*H*-)
υₘₐₓ cm⁻¹ 3442 (OH), 1558 (NO₂), 1376 (NO₂), 1521 (C=N), 1341 (OH), 1301 (OH), 1143 (C-N), 1176 (C-O), 1461 (Ar-H), 756 (C-Cl).

### 2. Synthèse du 2-(2'-Nitro-5'-chloro-phényl) benzoxazole (VLS269)

A du 2-((3'-Chloro-6'-nitro-benzylidène)-amino)-phénol agité dans du dichlorométhane à température ambiante a été ajouté 1,14g (0,005 mol) de 2,3-Dichloro-5,6-dicyano-1,4-benzoquinone (DDQ) sur une période de 5 minutes. Le mélange a été laissé à agiter durant 2 jours, filtré et évaporé afin d'obtenir le produit. Une masse de 0,97g d'une poudre verte/marron a été obtenue avec un rendement de 70% et un point de fusion mesuré de 130-132°C.

Les données qui ont été obtenues en RMN sont indiquées ci dessous :
High-resolution M.S.E.I for C₁₃H₇ClN₂O₃ Calculated mass of molecular ion 274.0140 (M+H)⁺ Measured mass: 274.0141. ¹H-NMR:(CDCl₃) δ 7.40-7.45 (2H, m, Ph-*H*), 7.56-7.61 (1H, m, Ph-*H*), 7.65 (1H, dd, J=8.66 Hz, J=2.23 Hz, 4'*H*), 7.80-7.85 (1H, m, Ph-*H*), 7.87 (1H, d, J=8.66 Hz, 5'*H*), 8.16 (1H, d, J=2.23 Hz, 2'*H*). υₘₐₓ cm⁻¹ 1534 (NO₂), 1371 (NO₂), 1572 (C=N), 1616 (C=N), 1182 (C-O), 1236 (C-O), 1153 (C-N), 1461 (Ar-H), 743 (C-Cl).

### 3. Synthèse du 2-(3',4'-Diméthoxy-6'-benzylidene)-amino)-phénol (VLS 260)

Un mélange de 3,27g (0,03 mol) de 2-hydroxyaniline et de 6,33g (0,03 mols) de 6-nitro-veratraldéhyde dans l'éthanol a été chauffé à reflux durant 3-4 heures. Le précipité cristallisé résultant a été collecté et séché dans un dessiccateur à pression réduite. Une masse de 8,20g de fins cristaux orange a été obtenue avec un rendement de 90% et un point de fusion mesuré de 196-198°C.

Les données qui ont été obtenues en RMN sont indiquées ci dessous :
¹H-NMR:(CDCl₃) δ . ¹H-NMR:(CDCl₃) δ 4.00 (3H, s, C*H*₃O), 6.92 (1H, t, J=7 Hz, Ph-*H*), 7.01 (1H, dd, J=7 Hz, J=1.4 Hz, Ph-*H*), 7.12 (1H, s (broad), O*H*), 7.22 (1H, t, J=7 Hz, Ph-*H*), 7.32 (1H, dd, J=7 Hz, J=1.4 Hz, Ph-*H*), 7.62 (1H, s, 5'*H*), 7.69 (1H, s, 3'*H*), 9.25 (1H, s, =C*H*-). υₘₐₓ cm⁻¹ 3416 (OH), 1566 (NO₂), 1384 (NO₂), 1589 (C=N), 1344 (OH), 1317 (OH), 1181 (C-N), 1149 (C-O), 1461 (Ar-H), 2838 (OMe).

### 4. Synthèse du 2-(2'-Nitro-4',5'-diméthoxy-phényl)-benzoxazole (VLS 261)

A 3,04g (0,01 mol) de 2-(3',4'-Diméthoxy-6'-benzylidene)-amino)-phénol agité dans du Dichlorométhane à température ambiante a été ajouté 2,27g (0,01 mol) de 2,3-Dichloro-5,6-dicyano-1,4-benzoquinone (DDQ) sur une période de 5 minutes. Le mélange a été laissé à agiter durant 2 jours, filtré et évaporé afin d'obtenir le produit. Une masse de 2,76g d'une poudre orange/marron a été obtenue avec un rendement de 90% et un point de fusion mesuré de 148-146°C.

Les données qui ont été obtenues en RMN sont indiquées ci dessous :
High-resolution M.S.E.I for C₁₅H₁₂N₂O₅ Calculated mass of molecular ion 301.0819 (M+H)⁺Measured mass: 301.0819. ¹H-NMR:(CDCl₃) δ 4.00 (6H, d, J=2.7 Hz, (OC*H*₃)₂), 7.37-7.41 (2H, m, Ph-*H*), 7.43 (1H, s, 5'*H*), 7.52 (1H, s, 2'*H*), 7.53-7.57 (1H, m, Ph-*H*), 7.78-7.82 (1H, m, Ph-*H*). υₘₐₓ cm⁻¹ 1519 (NO₂), 1378 (NO₂), 1582 (C=N), 1189 (C-N), 1177 (C-O), 1448 (Ar-H), 2850 (OMe).

### Substrat 7 - 2-(2'-Nitro-5'-fluoro-phényl)-benzoxazole - OF20B

Un mélange de 10,91g (0,100mol) de 2-Aminophénol, de 200ml d'éthanol et de 16,91g (0,100 mol) de 2-Nitro-5-fluorobenzaldéhyde a été chauffé par un bain d'huile à reflux à la température de 110°C durant une heure.

Le mélange a été refroidi à température ambiante et le précipité a été récupéré par filtration. Afin de récupérer un maximum de produit, le ballon a été rincé avec le filtrat pour évaporer une partie significative.

Le solide a été séché dans un dessicateur sous pression réduite pour obtenir 24,09g d'OF20A avec un rendement de 93%.

Le solide précédemment obtenu a été dissout dans 100 ml de Dichlorométhane, puis a été ajouté petit à petit 21,04g (0,0927mol) de 2,3-Dicyano-5,6-Dichloro-parabenzoquinone à température ambiante. La réaction s'est déroulée durant la nuit, la verrerie a été filtrée et rincée avec du D.C.M. afin de récupérer le maximum de produit puisque il est soluble dans le D.C.M.

Le solvant a été évaporé sous pression réduite afin d'obtenir 14,49g de OF20B, un solide marron-beige avec un rendement de 61%.

Les données qui ont été obtenues en RMN sont indiquées ci dessous :
¹H NMR (270 MHz, CDCl₃): δ = 7.37 (1H, d, *J* = 3 Hz, Ar-*H*), 7.43 (1H, t, *J* = 5 Hz, Ar-*H*), 7.45 (1H, s, Ar-*H*), 7.59 (1H, t, *J* = 5 Hz, Ar-*H*), 7.82 (1H, d, *J* = 3 Hz, Ar-*H*), 7.85 (1H, d, *J* = 3 Hz, Ar-*H*), 7.96 (1H, dd, *J* = 9 Hz, Ar-*H*), 8.15 (1H, d, *J* = 7 Hz, Ar-*H*).

### Substrat 3 : 2-(2'-Nitrophényl)-5-méthyl-benzoxazole

Le substrat No 3 a été synthétisé de façon analogue à la synthèse du 2-(2'-Nitrophényl)-benzoxazole (substrat No 1).

### Substrat 4 : 2-(2'-Nitro-5'-chloro-phényl)-benzoxazole

Le substrat No 4 a été synthétisé de façon analogue à la synthèse du 2-(2'-Nitrophényl)-benzoxazole (substrat No 1).

### Substrat 5 : 2-(2'-Nitro-4',5'-dimethylenedioxy-phenyl)-benzoxazole

Le substrat No 5 a été synthétisé de façon analogue à la synthèse du 2-(2'-Nitrophényl)-benzoxazole (substrat No 1).

### Substrat 8 : 2-(2'-Nitro-5'-(N-méthylpipérazinyl)-phényl)-benzoxazole (OF29A)

1,01g (0,010mol) d'N-Méthylpipérazine a été ajouté au mélange de 1,29g (0,005mol) de 2-(2'-Nitro-5'-fluorophényl)-benzoxazole et de 1,04g (0,0075mol) de K₂CO₃ dans 9 ml de Diméthysulfoxide. La réaction a été laissée sous agitation magnétique et chauffée à 90°C, en vérifiant son avancement par CCM.

Après achèvement de la réaction (au moins 8 heures) le mélange réactionnel a été dilué dans de l'acétate d'éthyle (77mL), la phase organique ainsi obtenue a été soigneusement lavée avec de 2 fois 65ml d'eau, puis avec 65ml de saumure et séchée à l'aide de sulfate de sodium afin d'obtenir 1,23g de pétales jaunes/dorées d'OF29A avec un rendement de 73%.

On peut effectuer une purification par chromatographie sur colonne si nécessaire, cela n'a pas été le cas pour nous.
¹H NMR (270 MHz, CDCl₃): δ = 2.35 (3H, s, C*H*₃), 2.57 (2H, t, *J* = 7 Hz, C*H*₂-CH), 3.48 (2H, t, *J* = 7 Hz, C*H*₂-CH), 6.95 (1H, d, *J* = 7 Hz, Ar-*H*), 6.98 (1H, d, *J* = 7 Hz, Ar-*H*), 7.21 (1H, d, *J* = 7 Hz, Ar-*H*), 7.28 (1H, s, Ar-*H*), 7.39 (1H, t, *J* = 9 Hz, Ar-*H*), 7.41 (1H, t, *J* = 7 Hz, Ar-*H*), 7.55 (1H, t, *J* = 7 Hz, Ar-*H*), 7.61 (1H, t, *J* = 7 Hz, Ar-*H*), 8.1 (1H, d, *J* = 7 Hz, Ar-*H*).
m.p.: 122-126
LC-MS-DI m/z Found: 339.54 (C₁₈H₁₈N₄O₃) (M+H): requires M. 338.37.

### • X est NX1-CO

### Substrat 20 - 2-(2'-Nitro-5'-chloro-phényl)-quinoxazol-4-one

### 1. Synthèse de la 1,2-Dihydro-2-(2'-nitro-5'-chloro-phényl)-quinoxazoline-4-one (VLS 276)

Une masse de 2,31g (0,017mol) de 2-Amino benzamide et 3,14g (0,017mol) de 3-Chloro-6-nitro benzaldéhyde a été chauffée dans l'éthanol à reflux durant 1 heure. Le mélange a ensuite été refroidi, et les cristaux oranges obtenus on été récoltés et séchés dans un dessiccateur à pression réduite. Une masse de 3,97g de cristaux orange clair a été obtenue avec un rendement de 76% et un point de fusion mesuré de 234-236°C. Les données qui ont été obtenues en RMN sont indiquées ci dessous :
¹H-NMR:(DMSO) δ 6.35 (1H, t, J=2 Hz, C*H*), 6.70 (1H, d, J=1 Hz, Ph-*H*(8*H*)), 6.80 (1H, t, J=7 Hz, Ph-*H* (6*H*)), 7.05 (1H, s (broad), N*H*), 7.25 (1H, t, J=7 Hz, Ph-*H* (7*H*)), 7.65 (1H, d,d, J=6 Hz, J=1.5 Hz, Ph-*H* (5*H*)), 7.75 (1H, d,d, J=9 Hz, J=3 Hz, Ph-H (4'*H*)), 7.85 (1H, d, J=3 Hz, Ph-*H* (6'*H*)), 8.15 (1H, d, J=9 Hz, Ph-H (3'*H*)), 8.30 (1H, d (broad), N*H*-CO), High-resolution M.S.E.I for C₁₄H₁₀ClN₃O₃ Calculated mass of molecular ion 304.0483 (M+H)⁺. Measured mass: 304.0482
υₘₐₓ cm⁻¹ 1661 (C=ONH), 1564 (NO₂), 1358 (NO₂), 1602 (C=N), 774 (C-Cl),

### 2. Oxydation de la 2-(2'-Nitro-5'-chloro-phényl)-quinoxazoline-4-one (VLS 278)

Une masse de 9,10g (0,03 mol) de 2-(2'-Nitro-5'-chloro-phényl)-quinoxazoline-4-one a été dissoute dans 40ml d'acétone anhydre et a été traitée par 4,74g (0,03 mol) d'une solution de Permanganate de potassium dans de l'acétone anhydre. Cette solution a été ajoutée goutte à goutte au mélange sur une période de 2-3 heures à température ambiante. Ensuite la réaction a été laissée sous agitation durant la nuit. L'excès de KMnO₄ a été éliminé par l'addition de bisulfite de sodium solide en excès. Puis la solution a été filtrée et évaporée. Une masse de 2,26g d'une poudre blanche et pâle a été obtenue avec un rendement de 25% et un point de fusion mesuré de 276-278°C.

Les données qui ont été obtenues en RMN sont indiquées ci dessous :
High-resolution M.S.E.I for C₁₄H₈ClN₃O₃ Calculated mass of molecular ion 302.0327 (M+H)⁺. Measured mass: 304.0327. ¹H-NMR:(DMSO) δ 7.55 (1H, t, J=7 Hz, Ph-*H* (7*H*)), 7.65 (1H, d, J=8 Hz, Ph-*H* (4'*H*)), 7.85 (1H, t, J=7 Hz, Ph-*H* (6*H*)), 7.90 (1H, d,d, J=8 Hz, J=2 Hz, Ph-*H* (5*H*)), 8.05 (1H, d, J=2 Hz, Ph-*H* (6'*H*), 8.25 (1H, d, J=8 Hz, Ph-*H* (3'*H*). υₘₐₓ cm⁻¹ 3132 (NH₂), 3070 (NH₂), 1578 (NO₂), 1368 (NO₂), 1603 (NH), 1660 (CONH), 1531 (C=N), 772 (C-Cl)

### Substrat 17 - 2-(2'-Nitrophényl)-3-méthyl-quinoxazol-4-one

### 1. Synthèse du 2-Amino-N-méthylbenzamide (VLS 291)

A 16,20g (0,099 mol) d'anhydride isotoique, 25ml méthylamine à 40% ont été ajoutés petit a petit sous agitation tout en contrôlant le dégagement de CO₂. Le mélange a été maintenu à température ambiante durant 1 heure. Ensuite le mélange a été neutralisé avec une solution de 2M d'HCl, filtré sous pression réduite avec un Buchi, lavé plusieurs fois à l'eau et séché à l'aide d'un dessiccateur à pression réduite. Une masse de 11,61g d'une poudre grise a été obtenue avec un rendement de 78%.

### 2. Synthèse de la 2-(2'-Nitro-5'-chloro-phényle)-3-méthyle-quinoxazoline-4-one (VLS 292)

Une masse de 7,5g (0,05 mol) de 2-Amino-*N*-méthyle benzamide et 9,30g (0,05 mol) de 3-Chloro-6-nitro benzaldéhyde ont été agités magnétiquement et portés à reflux durant 2-3 heures. Les cristaux oranges ainsi obtenus ont été filtrés sous pression réduite avec un Buchi et séchés à l'aide d'un dessiccateur à pression. une masse de 10,38g de cristaux oranges fins comme des aiguilles a été obtenue avec un rendement de 66%.

### 3. Oxydation de VLS 292 (VLS 295b)

Une masse de 3,17g (0,01 mol) de VLS 292b ont été dissous dans de l'acétone anhydre. 6,32g (0,04 mol) de KMnO₄ a été dissous dans de l'acétone anhydre et ajoutée goutte à goutte au précédent mélange sur une période d'1 à 2 heures. Puis la réaction a été laissée sous agitation magnétique durant la nuit. Ensuite le mélange a été neutralisé par du metabisulphite de sodium, filtré et évaporé pour enlever l'excès d'acétone. Une masse de 2,60g d'une poudre blanche a été obtenue avec un rendement de 80%.

### • X est NX1

### Substrat 14 - 2-(2'-Nitro-4',5'-cyclomethylenedioxy-phenyl)-3-phenethyl-benzimidazole (ou 2-(2'-Nitro-4',5'-méthylènedioxy-phényl)-3-phénéthyl-benzimidazole 1. N-Phényléthyl-2-nitroaniline (VLS 209)

Une masse de 4,8g (0,04 mol) de 2-Phényléthylamine a été ajoutée goutte à goutte, et ce à température ambiante, à un mélange de 5,6g (0,04 mol) de 2-Fluoronitrobenzène et de 11,06g (0,08 mol) de carbonate de potassium anhydre dans 30ml de DMSO. Le mélange a été chauffé à 100°C durant 3 heures. Ensuite, le mélange a été refroidi à température ambiante et a été ajouté à 100ml d'eau, ce qui a conduit à la précipitation d'un composé orange clair. Le solide a été récupéré par filtration et séché à l'aide d'un dessiccateur à pression réduite. Une masse de 9,4g de cristaux orange a été obtenue avec un rendement de 98%. Le point de fusion trouvé a été cohérent avec la valeur théorique, soit 64-66°C. Les données qui ont été obtenues en RMN sont indiquées ci dessous :
¹H-NMR: (CDCl₃) δ 3.00 (2H, t, J=7 Hz, -C*H*₂-) δ 3.55 (2H, t, J=7 Hz, -C*H*₂-), δ 6.65 (1H, m, Ph-*H*), δ 6.85 (1H, dd, J=8 Hz, J=2 Hz, Ph-*H*), δ 7.20-7.50 (6H, m, Ph-*H*), δ 8.10 (1H, s(broad), N*H*), δ 8.20 (1H, dd, J=8 Hz, J=2 Hz, Ph-*H*).

### 2. N-Phényléthyl-1,2-diaminobenzene (VLS 210)

A une suspension de 0,52g (0,002 mol) d'acétonate d'acétyle de cuivre dans l'éthanol, une solution de 0,38g (0,01 mol) de borohydrure de sodium a été ajoutée, agitée magnétiquement sous Azote à température ambiante. A cette solution ont été ajouté 2,42g (0,01 mol) du composé nitro VLS209 dans l'éthanol suivi de 0,76g (0,02 mol) de borohydrure de sodium dans l'éthanol. Le mélange a été laissé à agiter durant la nuit sous Azote à température ambiante. Ensuite le mélange a été concentré afin de retirer l'excès d'éthanol et de l'eau a été ajoutée. Puis, une extraction a été réalisée avec 2x20ml de DCM et la phase organique a été lavée plusieurs fois avec de l'eau, séchée à l'aide de carbonate de potassium et concentrée à l'évaporateur rotatif. Une masse de 1,69g d'un liquide sombre et huileux qui s'est solidifié par la suite a été obtenue, avec un rendement de 80%. Le point de fusion trouvé a été cohérent avec la valeur théorique, soit 42-44°C.

Les données qui ont été obtenues en RMN sont indiquées ci dessous :
¹H-NMR: (CDCl₃) δ 2.95 (2H, t, J=7 Hz, -C*H*₂-), δ 3.27 (2H, s (broad), N*H*₂), δ 3.38 (2H, t, J=7 Hz, -C*H*₂), δ 6.69 (3H, m, Ph-*H*), δ 6.82 (1H, m, Ph-*H*), δ 7.10-7.40 (5H, m, Ph-*H*).

### 3. 2-(2'-Nitro-4',5'-cyclomethylenedioxy-phenyl)-3-phenethyl-benzimidazole (VLS 213) (2-(2'-Nitro-4',5'-méthylènedioxy-phényl)-3-phénéthyl-benzimidazole

A une solution sous agitation magnétique de 5,08g (0,024 mol) de *N*-Phényléthyl-1,2-diaminobenzène, de 4,8g (0,024 mol) de 6-Nitropiperonal dans 25ml de DMF et d'1ml d'eau, 8,60g (0,014 mol) d'oxone (monopersulphate de potassium) ont été ajoutés durant une période de 15 minutes à température ambiante. Le mélange a été refroidi par un bain d'eau car la réaction était exothermique, et laissé à agiter durant la nuit. Ensuite de l'eau a été ajoutée au mélange et deux extractions consécutives au DCM ont été réalisées. Les phases organiques réunies ont été lavées à l'eau plusieurs fois, séchées (MgSO₄) et concentrées pour donner le produit brut d'une couleur jaune/marron. Une recristallisation avec de l'éthanol a été réalisée afin de purifier le composé. Une masse de 6,19g de cristaux marron a été obtenue avec un rendement de 67%, et un point de fusion mesuré de 162-164°C.

Les données qui ont été obtenues en RMN sont indiquées ci dessous :
High-resolution M.S.E.I for C₂₂H₁₇N₃O₄ Calculated mass of molecular ion 388.1292 (M+H)⁺. Measured mass: 388.1297. ¹H-NMR: (CDCl₃) δ 3.10 (2H, t, J=7 Hz,-C*H*₂-), δ 4.20 (2H, t, J=7 Hz,-C*H*₂-) δ 6.02 (1H, s, Ph-*H*), δ 6.18 (2H, s, O-C*H*₂-O), δ 6.80 (2H, dd, J=8 Hz, J=2 Hz, Ph-*H*), δ 7.10-7.40 (5H, m, Ph-*H*), δ 7.48 (1H, m, Ph-*H*), δ 7.65 (1H, s, Ph-*H*), δ 7.80 (1H, m, Ph-*H*).
υₘₐₓ cm⁻¹ 1364 (NO₂), 1523 (NO₂), 1610 (C=N), 1152 (C-N), 1207 (C-N), 1089 (C-O), 1473 (C-H), 728 (-CH₂).

### Substrat 12 - 2-(2'-Nitrophényl)-3-phénéthyl-benzimidazole - 54

Le substrat No 12 a été synthétisé de façon analogue à la synthèse du 2-(2'-Nitro-4',5'-cyclométhylènedioxy-phényl)-3-phenéthyl-benzimidazole (substrat No 14).

### 2 - Utilisation de substrats (substrats No 1 ; 2 ; 3 ; 4 ; 5 ; 6 ; 7 ; 8 ; 15 ; 12 ; 21 ; 23) pour détecter une activité nitroréductase dans des milieux selon l'invention.

### a) Préparation du milieu

Une masse de 40 mg de chaque substrat a été dissoute dans 4ml de Diméthyl formamide (DMF). Cette solution était intégralement reprise dans 400ml de gélose Columbia préalablement autoclavée et maintenue en surfusion à 50°C. La concentration finale pour chaque substrat était de 100mg/l.

Chacun des milieux était réparti en boîte de Petri de 90mm de diamètre à raison de 20ml par boîte.

### b) Ensemencement et incubation

18 souches de micro-organismes issues de collections et appartenant à différentes espèces de bactéries et de levures ont été ensemencées sur ces milieux par isolement semi-quantitatif de 10µl d'une suspension à 0,5 McFarland diluée au 20^{e}. Les milieux ont été incubés 24 heures à 37°C, puis les colonies formées ont été examinées visuellement sous éclairage UV à 360-365nm.

### c) Lecture des résultats

Les résultats obtenus sont présentés dans les tableaux 2 à 5.

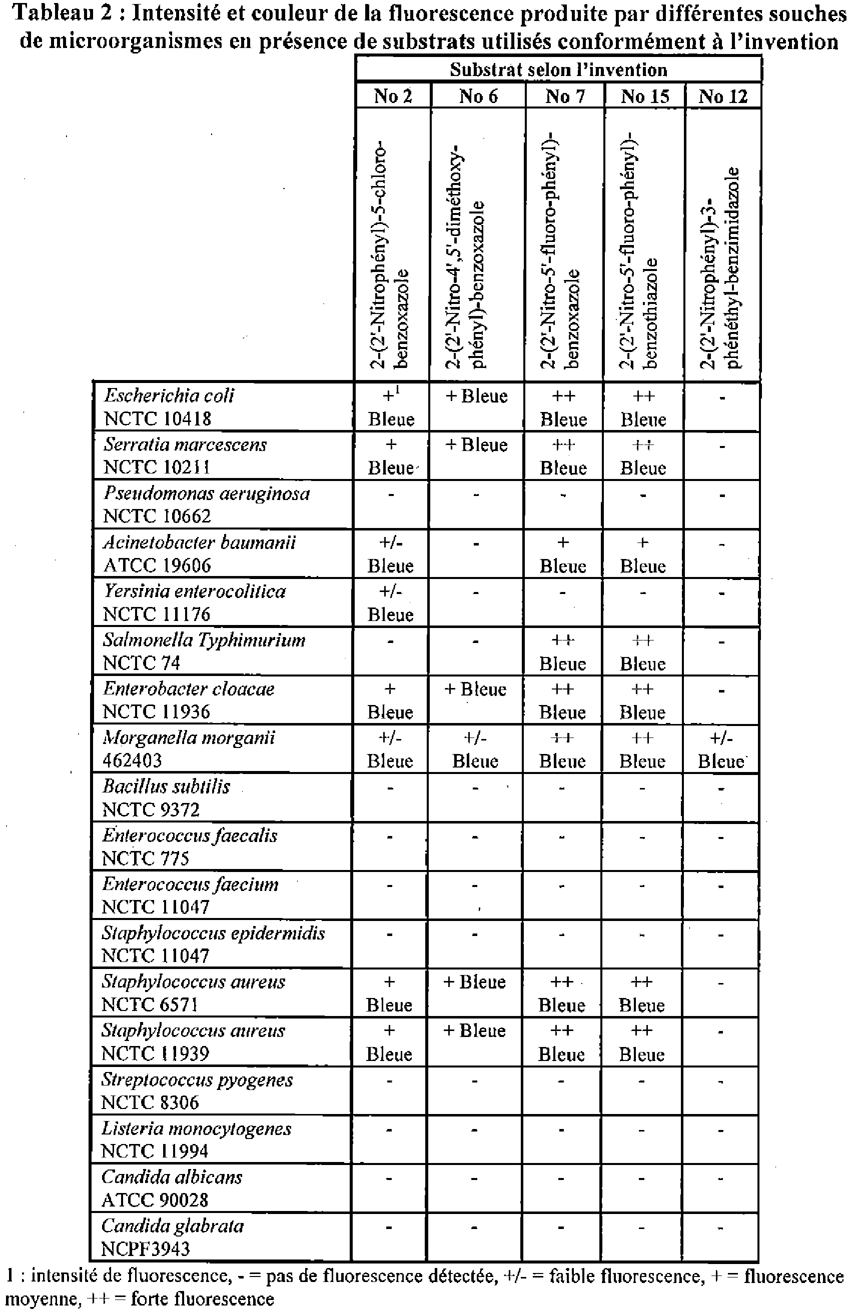

**Tableau 3 : Croissance, couleur et fluorescence produite par différentes souches de microorganismes en présence de substrats utilisés conformément à l'invention**

| | Substrat 1 | | Substrat 5 | |
|---|---|---|---|---|
| | Cr | F | Cr | F |
| *Escherichia coli* NCTC 10418 | + | ++ violet | + | ++ violet |
| *Serratia marcescens* NCTC 10211 | + | ++ violet | + | ++ violet |
| *Pseudomonas aeruginosa* NCTC 10662 | + | ++ violet | + | + violet |
| *Burkholderia cepacia* 1222 | + | ++ violet | + | + violet |
| *Yersinia enterocolitica* NCTC 11176 | + | ++ violet | + | - |
| *Salmonella typhimurium* NCTC 74 | + | ++ violet | ++ | ++ violet |
| *Citrobacter freundii* 46262 (wild) | + | ++ violet | + | ++ violet |
| *Morganella morganii* 462403 (wild) | + | ++ violet | + | ++ violet |
| *Enterobacter cloacae* NCTC 11936 | + | ++ violet | + | ++ violet |
| *Providencia rettgeri* NCTC 7475 | + | ++ violet | + | + violet |
| *Bacillus subtilis* NCTC 9372 | + | - | + | - |
| *Enterococcus faecails* NCTC 775 | + | ++ violet | + | - |
| *Enterococcus faecium* NCTC 7171 | + | + violet | + | - |
| *Staphylococcus epidermidis* NCTC 11047 | + | + violet | + | - |
| *Staphylococcus aureus* NCTC 6571 | + | ++ violet | + | ++ violet |
| MRSA NCTC 11939 | + | ++ violet | + | ++ violet |
| *Streptococcus pyogenes* NCTC 8306 | + | + violet | + | - |
| *Listeria monocytogenes* NCTC 11994 | + | ++ violet | + | + violet |
| *Candida albicans* ATCC 90028 | +/- | - | + | - |
| *Candida glabrata* NCPF 3943 | Pd C | - | +/- | - |

| | | | | |
|---|---|---|---|---|
| Cr : croisssance- F : fluorescence | | | | |

**Tableau 4 - Intensité et couleur de la fluorescence produite par différentes souches de microorganismes en présence de substrats utilisés conformément à l'invention**

| | **Substrat 15** | **Substrat 23** |
|---|---|---|
| *Escherichia coli* NCTC 10418 | ++ turquoise | - |
| *Serratia marcescens* NCTC 10211 | ++ turquoise | + vert |
| *Pseudomonas aeruginosa* NCTC 10662 | + turquoise | + jaune |
| *Acinetobacter baumanii* ATCC 19606 | + turquoise | - |
| *Yersinia enterocolitica* NCTC 11176 | - | - |
| *Salmonella typhimurium* NCTC 74 | ++ turquoise | +jaune |
| *Citrobacter freundii* 46262 (wild) | ++ turquoise | + jaune |
| *Morganella morganii* 462403 (wild) | ++ turquoise | + jaune |
| *Enterobacter cloacae* NCTC 11936 | ++ turquoise | + jaune |
| *Providencia rettgeri* NCTC 7475 | ++ turquoise | + jaune |
| *Bacillus subtilis* NCTC 9372 | - | - |
| *Enterococcus faecails* NCTC 775 | - | - |
| *Enterococcus faecium* NCTC 7171 | - | - |
| *Staphylococcus epidermidis* NCTC 11047 | - | - |
| *Staphylococcus aureus* NCTC 6571 | ++ turquoise | Tr. jaune |
| MRSA NCTC 11939 | ++ turquoise | +/- jaune |
| *Streptococcus pyogenes* NCTC 8306 | - | - |
| *Listeria monocytogenes* NCTC 11994 | - | - |
| *Candida albicans* ATCC 90028 | - | - |
| *Candida glabrata* NCPF 3943 | - | - |

| | | |
|---|---|---|
| 1 : intensité de fluorescence, - = pas de fluorescence détectée, +/- = faible fluorescence, + = fluorescence moyenne, ++ = forte fluorescence | | |

**Tableau 5 -Détection de différentes souches de S. aureus en présence de substrats utilisés conformément à l'invention**

| | Subtrat 2 | | Su btrat 3 | | Subtrat 4 | | Subtrat 6 | | Subtrat 7 | | Subtrat 8 | | Subtrat 21 | | Subtrat 15 | |
|---|---|---|---|---|---|---|---|---|---|---|---|---|---|---|---|---|
| | 24h | 48h | 24h | 48h | 24h | 48h | 24h | 48h | 24h | 48h | 24h | 48h | 24h | 48h | 24h | 48h |
| | Nb de souches détectées | | Nb de souches détectées | | Nb de souches détectées | | Nb de souches détectées | | Nb de souches détectées | | Nb de souches détectées | | Nb de souches détectées | | Nb de souches détectées | |
| *S. aureus (dont MRSA)* | 10 | 10 | 10 | 10 | 10 | 10 | 10 | 10 | 10 | 10 | 10 | 10 | 10 | 10 | 10 | 10 |
| 10 souches | | | | | | | | | | | | | | | | |
| *S. epidermidis* | 0 | 2 | 0 | 2 | 0 | 2 | 0 | 2 | 0 | 2 | 0 | 2 | 0 | 2 | 0 | 2 |
| 2 souches | | | | | | | | | | | | | | | | |
| *Staph spp* | 12 | 12 | 12 | 12 | 12 | 12 | 12 | 12 | 12 | 12 | 12 | 12 | 12 | 12 | 12 | 12 |
| 12 souches | | | | | | | | | | | | | | | | |
| *Enterococcus* | 0 | 1 | 0 | 1 | 0 | 1 | 0 | 1 | 0 | 1 | 0 | 1 | 0 | 1 | 0 | 1 |
| 6 souches | | | | | | | | | | | | | | | | |

Contrairement à ce qui est observé avec des substrats à base de 7-Nitro-coumarine (AMC) telle que l'acide 3-Carboxylique7-Nitro-coumarine, pour tous les substrats selon l'invention testés, la fluorescence était localisée au niveau de la colonie uniquement ou à proximité immédiate.

Ces résultats montrent que les milieux comprenant les substrats No 2, 3, 4, 6, 7, 8, 15, 21, permettent de différencier ou identifier les souches de *Staphylococcus aureus,* notamment les souches de MRSA (methicilline résistant *Staphylococcus aureus*) de la plupart des autres souches de bactéries à Gram positif. Du fait de l'importance médicale de cette espèce, cela peut être très utile pour la détection rapide et spécifique de *S. aureus* dans les prélèvements cliniques, mais également dans les prélèvements alimentaires.

Par ailleurs, en fonction des substituants sur les noyaux aromatiques, la spécificité des milieux selon l'invention vis-à-vis des bactéries à Gram négatif est variable. A titre d'exemple, les substrats 2, 7, 15 permettent de différencier les *Pseudomonas aeruginosa* des *Acinetobacter baumanii*, les *Salmonella Typhimurium* sont fortement positives avec les substrats 7 et 15 et négatives avec les substrats 2, 6 et 12. Le substrat 12 est spécifique de *Morganella morganii*.

### d) Conclusion

Les milieux selon l'invention sont très utiles pour la détection, la numération et/ou l'identification de micro-organismes d'intérêt médical, industriel ou environnemental.

## Revendications

1. Utilisation d'un substrat enzymatique de formule (I) suivante : 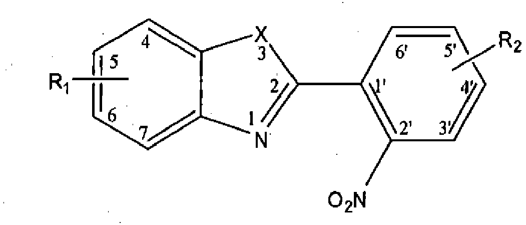 selon laquelle
• X représente NX1 ; O, NX1-CO,
• R1 représente rien, ou un substituant choisi parmi Cl, CH₃, Br, F, I, alkyle, aryle, carboxyle
• R2 représente rien ou un substituant choisi parmi Cl ; O-CH₂-O ; O-CH₃ ; F, diéthylènediamine-CH₃, NR3R4, Br, I, alkyle, aryle, carboxyle, NO₂ ; 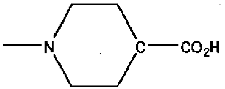
• R3 et R4 représentent indépendamment H ou un groupement alkyle ayant de 1 à 4 atomes de carbone
• X1 est choisi parmi H, CH₃, C₂H₄Ph, OH, alkyle, aryle
pour la détection chez des microorganismes, d'une activité nitroréductase.

2. Utilisation selon la revendication 1, pour la de détection et/ou l'identification de microorganismes.

3. Utilisation selon la revendication 2, en association avec un autre substrat enzymatique, spécifique d'une activité enzymatique différente d'une activité nitroréductase.

4. Procédé pour la détection chez des micro-organismes d'au moins une activité nitroréductase, **caractérisé en ce qu'**il comprend les étapes suivantes :
a) disposer d'un milieu de détection et/ou d'identification comprenant un substrat enzymatique de formule (I) suivante : 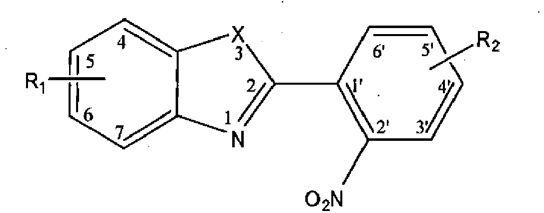 selon laquelle
• X représente NX1; O, NX1-CO,
• R1 représente rien, ou un substituant choisi parmi Cl, CH₃, Br, F, I, alkyle, aryle, carboxyle
• R2 représente rien ou un substituant choisi parmi Cl ; O-CH₂-O ; O-CH₃ ; F, diéthylènediamine-CH₃, NR3R4, Br, I, alkyle, aryle, carboxyle, NO₂ ; 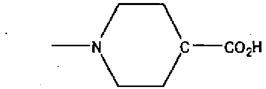
• R3 et R4 représentent indépendamment H ou un groupement alkyle ayant de 1 à 4 atomes de carbone
• X1 est choisi parmi H, CH₃, C₂H₄Ph, OH, alkyle, aryle ;
b) ensemencer le milieu avec un échantillon biologique à tester,
c) laisser incuber, et
d) révéler la présence d'au moins une activité nitroréductase.

## Patentansprüche

1. Verwendung eines Enzymsubstrats der folgenden Formel (I): 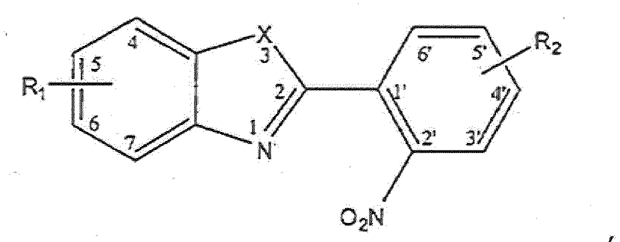 in der
• X NX1; O, NX1-CO bedeutet,
• R1 nichts oder einen Substituenten, ausgewählt aus Cl, CH₃, Br, F, I, Alkyl, Aryl, Carboxyl, bedeutet,
• R2 nichts oder einen Substituenten, ausgewählt aus Cl; O-CH₂-O; O-CH₃; F, Diethylendiamin-CH₃, NR3R4, Br, I, Alkyl, Aryl, Carboxyl, NO₂; 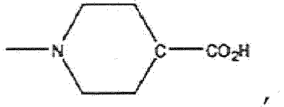 bedeutet,
• R3 und R4 unabhängig voneinander H oder eine Alkylgruppe mit 1 bis 4 Kohlenstoffatomen bedeuten,
• X1 aus H, CH₃, C₂H₄Ph, OH, Alkyl, Aryl ausgewählt ist,
für den Nachweis einer Nitroreduktaseaktivität bei Mikroorganismen.

2. Verwendung nach Anspruch 1 für den Nachweis und/oder die Bestimmung von Mikroorganismen.

3. Verwendung nach Anspruch 2 zusammen mit einem weiteren Enzymsubstrat, das für eine Enzymaktivität, die sich von einer Nitroreduktaseaktivität unterscheidet, spezifisch ist.

4. Verfahren zum Nachweis von mindestens einer Nitroreduktaseaktivität bei Mikroorganismen, **dadurch gekennzeichnet, dass** es die folgenden Schritte umfasst:
a) Verfügen über ein Nachweis- und/oder Bestimmungsmedium umfassend ein Enzymsubstrat der folgenden Formel (I): 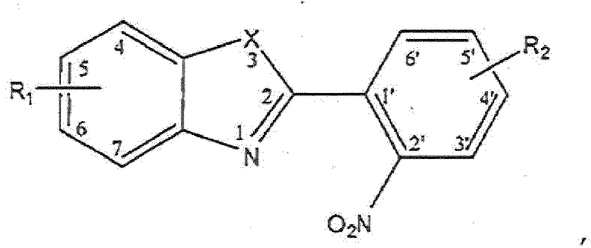 in der
• X NX1; O, NX1-CO bedeutet,
• R1 nichts oder einen Substituenten, ausgewählt aus Cl, CH₃, Br, F, I, Alkyl, Aryl, Carboxyl, bedeutet,
• R2 nichts oder einen Substituenten, ausgewählt aus Cl; O-CH₂-O; O-CH₃; F, Diethylendiamin-CH₃, NR3R4, Br, I, Alkyl, Aryl, Carboxyl, NO₂; 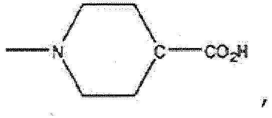 bedeutet,
• R3 und R4 unabhängig voneinander H oder eine Alkylgruppe mit 1 bis 4 Kohlenstoffatomen bedeuten,
• X1 aus H, CH₃, C₂H₄Ph, OH, Alkyl, Aryl ausgewählt ist,
b) Inokulieren des Mediums mit einer biologischen Testprobe,
c) Inkubierenlassen und
d) Aufzeigen des Vorhandenseins von mindestens einer Nitroreduktaseaktivität.

## Claims

1. A use of an enzyme substrate of formula (I) below: 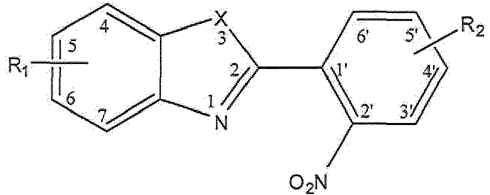 in which:
• X is NX1, 0 or NX1-CO;
• R1 is nothing or a substituent selected from Cl, CH₃, Br, F, I, alkyl, aryl and carboxyl;
• R2 is nothing or a substituent selected from Cl, O-CH₂-O, O-CH₃, F, diethylenediamine-CH₃, NR3R4, Br, I, alkyl, aryl, carboxyl, NO₂ and 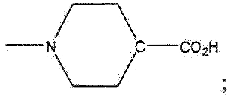
• R3 and R4 are independently H or an alkyl group containing from 1 to 4 carbon atoms;
• X1 is selected from H, CH₃, C₂H₄Ph, OH, alkyl and aryl;
for detecting nitroreductase activity, in microorganisms.

2. The use as claimed in claim 1, for detecting and/or identifying microorganisms.

3. The use as claimed in claim 2, in addition with at least one other enzyme substrate, specific for an enzymatic activity different to a nitroreductase activity.

4. A method for detecting at least one nitroreductase activity in microorganisms, **characterized in that** it comprises the following steps:
a) providing a detection and/or identification medium comprising an enzyme substrate of formula (I) below: 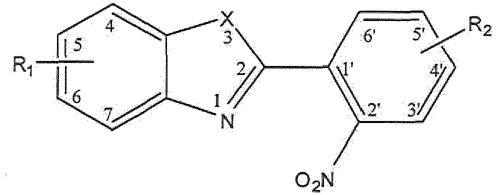 in which:
• X is NX1, O or NX1-CO;
• R1 is nothing or a substituent selected from Cl, CH3, Br, F, I, alkyl, aryl and carboxyl;
• R2 is nothing or a substituent selected from Cl, O-CH2-O, O-CH3, F, diethylenediamine-CH3, NR3R4, Br, I, alkyl, aryl, carboxyl, NO2 and 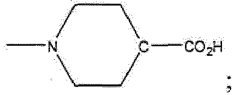
• R3 and R4 are independently H or an alkyl group containing from 1 to 4 carbon atoms;
• X1 is selected from H, CH3, C2H4Ph, OH, alkyl and aryl;
b) inoculating the medium with a biological sample to be tested,
c) leaving this to incubate, and
d) revealing the presence of at least one nitroreductase activity.
